# NEUE EUROPÄISCHE PATENTSCHRIFT

(11) **EP 1 157 677 B2**
(45) Veröffentlichungstag und Bekanntmachung des Hinweises auf die Entscheidung über den Einspruch: **23.05.2012**
(45) Hinweis auf die Patenterteilung: 14.02.2007
(21) Anmeldenummer: 01105912.8
(22) Anmeldetag: 09.03.2001
(51) Int. Cl.: A61F 2/46

(54) **Vorrichtung zum Applizieren von Knochenzement und Kanüle für eine solche Vorrichtung**
Device for applying bone cement and cannula for such a device
Dispositif pour appliquer du ciment osseux et canule pour un tel dispositif

(30) Priorität: 22.12.2000 DE 10064202; 25.05.2000 DE 10025898
(43) Veröffentlichungstag der Anmeldung: 28.11.2001
(60) Teilanmeldung: 06017467.9
(73) Patentinhaber: Pajunk GmbH & Co. KG Besitzverwaltung, 78187 Geisingen (DE)
(72) Erfinder: Pajunk, Heinrich, 78187 Geisingen (DE); Pajunk, Horst, 78187 Geisingen (DE)
(74) Vertreter: Manitz, Finsterwald & Partner GbR

(56) Entgegenhaltungen:
- EP-A- 1 074 231
- WO-A-99/65597
- WO-A1-90/03823
- DE-A- 19 532 015
- FR-A- 577 367
- US-A- 2 711 733
- US-A- 4 576 152
- US-A- 4 583 974
- US-A- 4 653 489
- US-A- 4 653 489
- US-A- 4 832 692
- US-A- 5 051 482
- US-A- 5 137 514
- US-A- 5 398 483
- US-A- 5 507 727
- US-A- 5 893 488
- US-A- 6 048 346
- AL-ASSIR ET AL.: 'Percutaneous vertebroplasty: A special syringe for cement injection' AJNR AM. J. NEURORADIOLOGIE Nr. 21, 21 Januar 2000, Seiten 159 - 161
- GANGI ET AL.: 'Intéret de l'injection percutanée de ciment acrylique a l'aide d'un manche de pression' JOURNAL DE RADIOLOGIE Nr. 78, 1997, PARIS, Seiten 393 - 394

## Beschreibung

Die vorliegende Erfindung betrifft eine Vorrichtung zum Applizieren von Knochenzement nach dem Oberbegriff des Anspruchs 1. Weiterhin wird eine Kanüle für eine Vorrichtung zum Applizieren von Knochenzement mit einer am distalen Ende ausgebildeten Öffnung und einem an proximalen Ende vorgesehenen Kopplungsabschnitt zum Ankoppeln an die Applikationsvorrichtung beschrieben.

Applikationsvorrichtungen dieser Art werden verwendet, wenn Knochenstrukturen beispielsweise durch Knochenkrebs oder durch Osteoporose zersetzt oder brüchig geworden sind. Mit entsprechenden Vorrichtungen ist die Applikation von Knochenzement unmittelbar in die betroffenen Knochenstrukturen möglich, wodurch die verfestigt werden.

Bei der Applikation sind dabei mehrere Anforderungen zu beachten. Zum einen muß die Befüllung der Applikationsvorrichtung sowie die Applikation in die betroffenen Knochenstrukturen sehr schnell, innerhalb weniger Minuten erfolgen, da die üblicherweise verwendeten Knochenzemente 6 bis 7 Minuten nach dem Anrühren auszuhärten beginnen. Zum anderen muß der Knochenzement mit sehr hohem Druck appliziert werden, da andernfalls eine ausreichende Durchdringung der Knochenstrukturen nicht gewährleistet ist. Letztlich muß die Applikation des Knochenzements gut steuerbar sein, da insbesondere bei der Applikation im Bereich der Wirbelsäule ein Fehlleiten des Knochenzements zu irreversiblen Schädigungen beispielsweise von Nerven führen kann.

Aus der WO 99/65597 A ist eine Vorrichtung zum Applizieren von Knochenzement bekannt, bei der ein in einem Zylinder angeordneter Kolben durch Schraubbewegung den Knochenzement unter hohem Druck durch eine Kanüle hinausdrückt. In der DE 195 32 015 A1 ist eine ähnliche Applikationsvorrichtung beschrieben. In der US 4,653,489 A ist eine Applikationsvorrichtung beschrieben, bei der der Knochenzement über eine übliche Spritzenanordnung appliziert wird.

Es ist eine Aufgabe der vorliegenden Erfindung, eine Vorrichtung zum Applizieren von Knochenzement sowie eine Kanüle für eine Applikationsvorrichtung anzugeben, mit denen die Applikation in kurzer Zeit durchgeführt werden kann, wobei gleichzeitig der erforderliche hohe Druck aufgebaut werden kann und eine Steuerbarkeit des applizierten Knochenzements möglich ist.

Diese Aufgabe wird ausgehend von einer Applikationsvorrichtung der eingangs genannten Art erfindungsgemäß durch die Merkmale des Anspruchs 1 gelöst. Die Vorrichtung kann eine Kanüle umfassen, welche dadurch gekennzeichnet ist, daß das distale Ende der Kanüle asymmetrisch mit einer seitlich der Mittelachse liegenden Spitze ausgebildet ist und daß am proximalen Ende der Kanüle seitlich nach außen ragende Angriffselemente vorgesehen sind, mit denen die Kanüle sowohl um ihre Längsachse verdrehbar als auch entlang ihrer Längsachse verschiebbar ist.

Im Rahmen dieser Anmeldung wird dabei der Begriff "proximal" mit der Bedeutung "zum Körper des Arztes hin gelegen" verwendet. Dementsprechend wird der Begriff "distal" mit der Bedeutung "vom Körper des Arztes entfernt gelegen" verwendet.

Durch die erfindungsgemäße Umstellbarkeit der Applikationsvorrichtung ist es möglich, daß beispielsweise das Auffüllen des Zylinders durch eine Aufziehbewegung des Kolbens, d.h. eine direkte Verschiebung des Kolbens in Längsrichtung, in sehr kurzer Zeit erfolgt. In umgekehrter Weise kann anschließend der im Zylinder vorhandene, flüssige Knochenzement durch direktes Vorschieben des Kolbens in kurzer Zeit so lange appliziert werden, bis der entstehende Gegendruck so groß wird, daß er durch die direkte Vorschubbewegung nicht mehr überwunden werden kann. In diesem Moment wird die Applikationsvorrichtung auf einen Modus "Verschiebung des Kolbens durch Schraubbewegung" umgestellt, da durch die Schraubbewegung ein wesentlich höherer Druck auf den Kolben und damit auf den zu applizierenden Knochenzement ausgeübt werden kann als mit einer direkten Vorschubbewegung.

Die Vorschubgeschwindigkeit ist bei der Verschiebung durch die Schraubbewegung zwar deutlich geringer als bei der direkten Verschiebung in Längsrichtung; da bis zum Erreichen des beschriebenen hohen Drucks jedoch sowohl das Auffüllen des Zylinders als auch das Applizieren des flüssigen Knochenzements durch die direkte Längsverschiebung des Kolbens in sehr kurzer Zeit erfolgen kann, ist üblicherweise zu dem Zeitpunkt, an dem auf die weitere Applizierung durch Schraubbewegung umgestellt werden muß, noch ausreichend Zeit vorhanden, um die Applikation zu beenden, bevor der Knochenzement auszuhärten beginnt.

Ein weiterer Vorteil der erfindungsgemäß ausgebildeten Applikationsvorrichtung besteht darin, daß der während der Applikation mit Schraubbewegung aufgebaute hohe Druck sehr schnell, d.h. innerhalb von Sekundenbruchteilen abgebaut werden kann. Dies ist beispielsweise dann erforderlich, wenn bei der Beobachtung des aus der Kanüle austretenden Knochenzements beispielsweise an einen Fluoroskop, eine Fehlleitung des Knochenzements erkannt wird. In diesem Fall kann durch einfaches Umstellen der Vorrichtung auf die direkte Längsverschiebbarkeit des Kolbens erreicht werden, daß durch den hohe Druck der Kolben zurückgeschoben und damit automatisch der hohe Druck abgebaut wird. Auf diese Weise wird der fehlgeleitete Austritt des Knochenzements aus dem distalen Ende der Kanüle unmittelbar gestoppt.

Nach einer vorteilhaften Ausführungsform der Erfindung umfaßt der Kolben einen Eingriffsabschnitt mit einem Schraubgewinde, das in eine am Gehäuse vorgesehene Gegenverzahnung eingreift, so daß beim Verdrehen des Eingriffsabschnitts die Längsverschiebung des Kolbens erfolgt. Auf diese Weise wird ein sehr einfacher, kostengünstiger und funktionssicherer Aufbau einer erfindungsgemäße Applikationsvorrichtung erreicht. Insbesondere wird in dem Betriebsmodus "Verschiebung durch Schraubbewegung" durch die ineinandergreifenden Verzahnungen automatisch eine direkte Verschiebung in Längsrichtung verhindert, so daß die mit jeder Umdrehung erzielter Erhöhung des angewendeten Drucks automatisch gesichert ist.

Nach einer weiteren bevorzugten Ausführungsform der Erfindung sind das Schraubgewinde und die Gegenverzahnung entkoppelbar. Dabei ist zur Entkopplung die Gegenverzahung in einer Richtung im wesentlichen senkrecht zur Verschieberichtung des Kolbens zwischen einer Verriegelungsstellung und einer Freigabestellung bewegbar. Durch diese Ausbildung ist eine einfache und schnelle Umschaltung von dem Betriebszustand "Verschieben durch Schraubbewegung" in den Betriebszustand "direkte Verschiebung in Längsrichtung" und zurück möglich, indem beispielsweise über eine am Gehäuse angebrachte Betätigungseinheit die Gegenverzahnung in die Freigabestellung verschoben wird.

Die Gegenverzahnung wird unter Vorspannung gegen das Schraubgewinde gedrückt. Dadurch ist gewährleistet, daß der aufgebaute Druck solange automatisch gesichert ist, bis die Gegenverzahnung entgegen der Vorspannung bewegt wird. Diese Vorspannung erfolgt durch eine Federbeaufschlagung.

Nach einer weiteren vorteilhaften Ausführungsform der Erfindung umgreift die Gegenverzahnung das Schraubgewinde bereichsweise, insbesondere hinterschneidungsfrei. Durch eine bereichsweise Umgreifung des Schraubgewindes wird eine vergrößerte Kontaktfläche zwischen den unter hohem Druck aneinander anliegenden Zähnen der Gegenverzahnung und dem Gewindeprofil des Schraubgewindes erreicht, so daß die Stabilität der Vorrichtung erhöht ist. Durch das hinterschneidungsfreie Umgreifen ist dabei gewährleistet, daß weiterhin eine einfache Entkopplung von Schraubgewinde und Gegenverzahnung, beispielsweise durch einfaches seitliches Verschieben der Gegenverzahnung möglich ist.

Bevorzugt ist im entkoppelten Zustand der Kolben im wesentlichen frei in dem Zylinder längsverschiebbar. Die freie Verschiebbarkeit des Kolbens ist dabei im wesentlichen nur durch eine Dichtung beeinträchtigt, die üblicherweise zwischen dem Kolbenumfang und der Innenwand des Zylinders zur Abdichtung vorgesehen ist.

Nach einer weiteren vorteilhaften Ausführungsform der Erfindung schlie-ßen die beim Applizieren des Knochenzements unter Druck aneinander anliegenden Zahnflanken der Gegenverzahnung und/oder die Flanken des Gewindeprofils des Schraubgewindes einen Winkel von kleiner oder gleich ca. 90° mit der zur Verschieberichtung parallel verlaufenden Längsachse des Eingriffsabschnitts ein. Durch diese spezielle Ausbildung der Flanken ist gewährleistet, daß auch bei Anwendung sehr hoher Drücke kein Überschnappen einzelner Zähne auftritt, wie es beispielsweise bei üblichen, abgeschrägten Flanken der Fall sein kann, bei denen der Winkel zwischen den Flanken und der Längsachse des Eingriffsabschnitts größer als 90° ist. Beträgt der Winkel im wesentlichen gleich 90°, so kann die Gegenverzahnung zum Entkoppeln von dem Schraubgewinde durch eine Schiebebewegung senkrecht zur Bewegungsrichtung des Eingriffsabschnitts verschoben werden. Sind die Winkel kleiner als 90°, so ist eine Entkopplung durch eine entsprechende Verschiebung der Gegenverzahnung schräg zur Längsachse des Eingriffsabschnitts möglich.

Bevorzugt sind der Kolben und der Eingriffsabschnitt einstückig ausgebildet. Bei einer einstückigen Ausbildung muß gewährleistet sein, daß der Kolben in dem Zylinder verdrehbar ist, um auf diese Weise die Verschraubung des Eingriffsabschnitts zu ermöglichen. Bei dieser Ausführungsform wird somit die Längsverschiebung des Kolbens unmittelbar durch Einschrauben des Kolbens erzielt.

Es ist auch möglich, daß der Kolben zweiteilig ausgebildet ist, so daß der Eingriffsabschnitt ein separates Teil bildet. In diesem Fall sind beide Teile miteinander insbesondere gegeneinander verdrehbar verbunden. Bei dieser Ausführung ist es möglich, daß bei der Schraubbewegung lediglich der Eingriffsabschnitt verdreht wird, während der Kolben unverdreht durch den sich vorwärtschraubenden Eingriffsabschnitt innerhalb des Zylinders nach vorne verschoben wird. Während in diesem Fall die Querschnittsflächen des Kolbens und des Zylinders zwar komplementär zueinander, in der Form jedoch grundsätzlich beliebig sein können, beispielsweise oval oder eine mehrkantige Form besitzen können, ist bei der einstückigen Ausbildung von Kolben und Eingriffsabschnitt der Zylinder üblicherweise als Kreiszylinder ausgebildet, um auf diese Weise eine Verdrehung des Kolbens im Zylinder zusammen mit dem Eingriffsabschnitt zu ermöglichen.

Nach einer weiteren vorteilhaften Ausführungsform der Erfindung ist an der Austrittsöffnung des Zylinders eine Kanüle befestigbar. Diese Kanüle ist bevorzugt lösbar befestigt, da auf diese Weise in einem ersten Verfahrensschritt die Kanüle ohne Applikationsvorrichtung in den Patienten eingebracht und positioniert werden kann, während erst nach erfolgreicher Plazierung in einem zweiten Verfahrensschritt die Applikationsvorrichtung beispielsweise über eine ebenfalls an der Austrittsöffnung befestigbaren Aufziehkanüle mit dem flüssigen Knochenzement befüllt wird. Nach Entfernen der Aufziehkanüle kann anschließend die Applikationsvorrichtung an der bereits eingesetzten Injektionskanüle befestigt werden und der Knochenzement in der zuvor beschriebenen Weise appliziert werden.

Die erfindungsgemäße Kanüle hat den Vorteil, daß aufgrund ihrer asymmetrischen Spitze sowie der seitlich nach außen ragenden Angriffselemente bereits beim Einbringen der Kanüle eine exakte Positionierung vorgenommen werden kann. Übliche Kanülen weisen eine symmetrische Spitze auf und können beim Einbringen bezüglich der Einbringrichtung nicht verändert werden. Bei der erfindungsgemäßen Kanüle kann hingegen die asymmetrische Spitze durch ein Verdrehen der teilweise eingeführten Kanüle über die seitlich nach außen ragenden Angriffselemente so positioniert werden, daß eine Bewegung der Kanüle beim weiteren Einbringen in die gewünschte Richtung erreicht wird. Durch die vorauseilende asymmetrische Spitze wird die Kanüle beim Einbringen immer geringfügig in die Richtung seitlich abweichen, zu der die Spitze gerade hin gelegen ist. Durch wiederholtes abwechselndes Einbringen und Verdrehen ist somit mit der erfindungsgemäß ausgebildeten Kanüle eine verbesserte Positionierung sowie ein nachträgliches Korrigieren der Position noch während des Einbringens möglich.

Weiterhin ist es mit der erfindungsgemäßen Kanüle möglich, nach Aushärtung des applizierten Knochenzements durch Verdrehen der noch im Körper befindlichen Kanüle über die Angriffselemente um ihre Längsachse ein Abdrehen bzw. Abscheren des sich noch innerhalb der Kanüle befindenden aushärtenden Knochenzements von dem in die Knochenstruktur applizierten Knochenzements zu ermöglichen. Dadurch ist sichergestellt, daß der sich innerhalb der Kanüle befindende Knochenzement beim Herausziehen der Kanüle in dieser verbleibt und sicher mit dieser zusammen aus dem Gewebe entnommen wird.

Bevorzugt ist die die Umrandung der Öffnung bildende Kante des Kanülenendes als Schneide geschliffen. Dadurch ist auch bei bereits vollständig ausgehärtetem Knochenzement eine sicher Abscherung des innerhalb der Kanüle angeordneten Materials gewährleistet.

Nach einer weiteren vorteilhaften Ausführungsform der Erfindung erstreckt sich die Durchtrittsfläche der Öffnung schräg zur Längsachse der Kanüle. Auf diese Weise kann die Bewegungsrichtung des aus der Öffnung in der Kanülenspitze austretenden Knochenzements gesteuert werden. Durch Verdrehen der Kanüle über die Angriffselemente kann die Öffnung so plaziert werden, daß das austretende Material in die gewünschte Richtung fließt. Bei Kanülen mit einer zentrischen Öffnung, wie sie aus dem Stand der Technik bekannt sind, tritt der Knochenzement hingegen immer in Längsrichtung nach vorne aus, so daß durch ein Verdrehen der Kanüle die Austrittsrichtung nicht geändert werden kann.

Weitere vorteilhafte Ausführungsformen der Erfindung sind in den Unteransprüchen angegeben.

Die Erfindung wird nachfolgend anhand eines Ausführungsbeispiels unter Bezugnahme auf die Zeichnungen näher beschrieben; in diesen zeigen:
- Fig. 1: einen teilweise aufgerissenen Querschnitt einer erfindungsgemäß ausgebildeten Applikationsvorrichtung,
- Fig. 2: eine Detailansicht einer Vorrichtung nach Fig. 1,
- Fig. 3: einen teilweise aufgerissenen Querschnitt der Vorrichtung nach Fig. 1,
- Fig. 4: eine erfindungsgemäß ausgebildete Kanüle,
- Fig. 5: eine Detailansicht der Kanüle nach Fig. 4,
- Fig. 6: eine weitere Ausführungsform einer erfindungsgemäß ausgebildeten Kanüle und
- Fig. 7: eine Detailansicht der Kanüle nach Fig. 6.

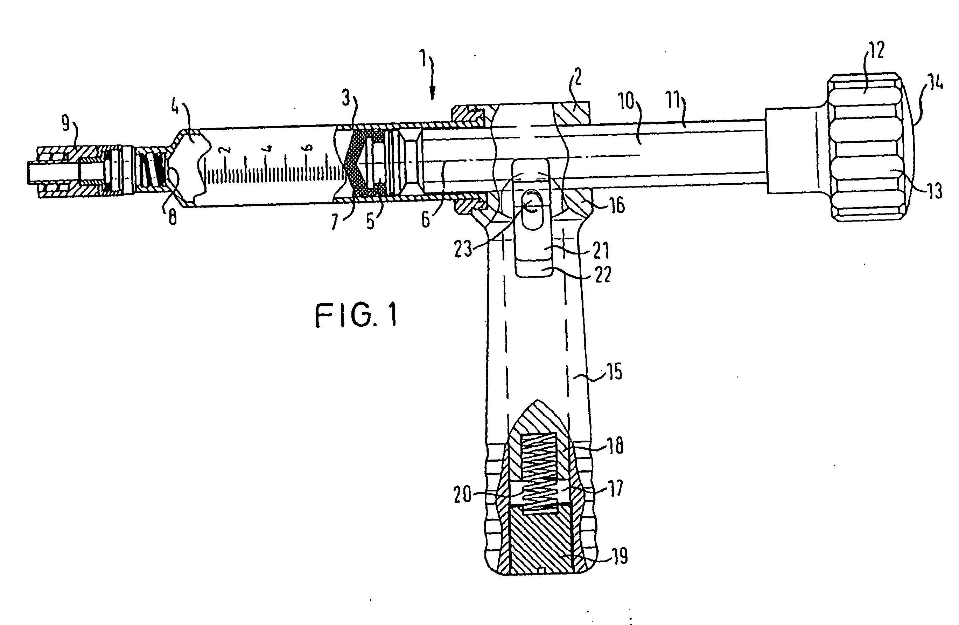

Fig. 1 zeigt eine pistolenförmige Applikationsvorrichtung 1 mit einem Gehäuse 2, dessen mittlerer Bereich als Zylinder 3 zur Aufnahme von Knochenzement in seinem Inneren 4 ausgebildet ist. Innerhalb des Zylinders 3 ist ein Kolben 5 in Richtung seiner Längsachse 6 verschiebbar gelagert, wobei der Kolben 5 mit einem Abdichtungsmittel 7 gegenüber der Innenwand des Zylinders 3 abgedichtet ist. Am distalen Ende des Zylinders 3 ist eine Austrittsöffnung 8 ausgebildet, an der ein beispielsweise als große Luer Lock-Verbindung ausgebildetes Kopplungselement 9 zum Ankoppeln einer Kanüle vorgesehen ist.

Der Kolben 5 ist einstückig mit einem langgestreckten Schaft 10 ausgebildet, der zum Bilden eines Eingriffsabschnitt an seiner Außenseite mit einem Schraubgewinde 11 versehen ist. Am proximalen Ende des Schaftes 10 ist ein als Dreh-/Schiebeknopf ausgebildetes Betätigungselement 12 vorgesehen, das an seiner Umfangsfläche Vertiefungen 13 und an seinem stirnseitigen Ende eine gewölbte Angriffsfläche 14 besitzt.

Das Gehäuse 2 umfaßt weiterhin einen sich in Fig. 1 nach unten erstrekkenden, einen Handgriff 15 bildenden Abschnitt, durch dessen oberen Bereich 16 der Schaft 10 des Kolbens 5 hindurchgeführt ist und der einen Hohlraum 17 umfaßt, in dem ein Verriegelungselement 18 längsverschiebbar angeordnet ist.

Der Hohlraum 17 ist zum freien Ende des Handgriffs 15 hin offen ausgebildet und dort mit einem beispielsweise eingeschraubten Verschlußelement 19 verschlossen. Das Verschlußelement 19 dient gleichzeitig als Abstützelement für eine Schraubenfeder 20, mit der das Verriegelungselement 18 in Richtung des oberen Bereichs 16 des Handgriffs 15 gedrängt wird, so daß es an dem Schaft 10 des Kolbens 5 zur Anlage kommt.

Mit dem Verriegelungselement 18 ist ein als Schieber ausgebildetes Entriegelungselement 21 verbunden, das in einer Führungsausnehmung 22 an der Außenseite des Gehäuses 2 verschiebbar geführt ist. Das Entriegelungselement ist über einen Bolzen 23 mit dem Verriegelungselement 18 so verbunden, daß bei einer Verschiebung des Entriegelungselements 21 in Richtung des freien Endes des Handgriffs 15 das Verriegelungselement 18 entgegen der Federkraft der Feder 20 verschoben wird.

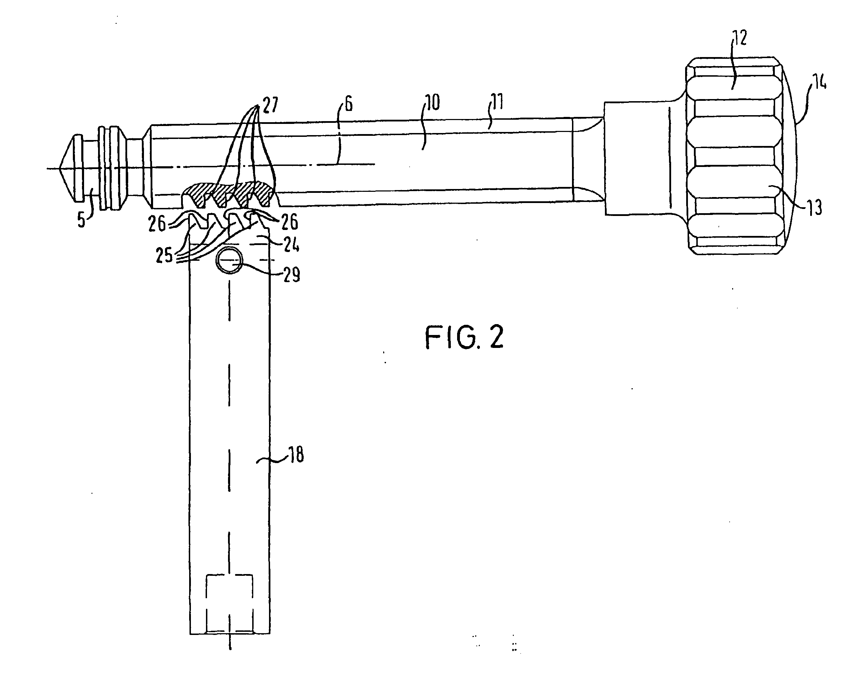

Das Zusammenwirken des Verriegelungselements 18 mit dem Schaft 10 des Kolbens 5 ist in Fig. 2 deutlicher dargestellt.

In Fig. 2 ist zu erkennen, daß das zum Schaft 10 hin gelegene Ende des Verriegelungselements 18 als Zahnstange 24 ausgebildet ist, dessen Zähne 25 eine Gegenverzahnung für das Schraubgewinde 11 des Schaftes 10 bilden.

Wird das Entriegelungselement 21 zum freien Ende des Handgriffs 15 nach unten verschoben, so wird über den Bolzen 23 das Verriegelungselement 18 entgegen der Kraft der Feder 20 nach unten verschoben, bis das Verriegelungselement 18 und der Schaft 10 entkoppelt sind, wie es in Fig. 2 dargestellt ist. In diesem Zustand kann der Schaft 10 und damit der Kolben 5 in dem Zylinder 3 direkt in Längsrichtung im wesentlichen frei verschoben werden, indem beispielsweise mit der Handfläche auf die Angriffsfläche 14 gedrückt wird. Lediglich durch die vorhandene Reibung zwischen dem Abdichtungsmittel 7 und der Innenwand des Zylinders 3 wird diese Verschiebbarkeit etwas beeinträchtigt.

Wird das Entriegelungselement 21 wieder freigegeben, so wird das Verriegelungselement 18 aufgrund der Kraft der Feder 20 in Richtung des Schafts 10 gedrängt, bis die Zähne 25 der Zahnstange 24 mit dem Schraubgewinde 11 des Schaftes 10 in Eingriff sind. In diesem Zustand ist eine Längsverschiebung des Schaftes 10 und damit des Kolbens 5 lediglich durch eine Verschraubung des Schafts 10 möglich, wobei dies bevorzugt über das als Schraubknopf ausgebildete Betätigungselement 12 erfolgt. In diesem Zustand erfolgt durch die Schraubbewegung zwar nur jeweils ein relativ geringer Vorschub des Kolbens 5, der durch die Schraubbewegung aufbringbare Druck auf den im Inneren 4 des Zylinders 3 angeordneten Knochenzement ist jedoch wesentlich höher als er durch eine direkte Verschiebung in Längsrichtung des Schaftes 10 bei von dem Verriegelungselement 18 entkoppelten Schaft 10 erzeugt werden kann.

Wie Fig. 2 zu entnehmen ist, sind die bei einem Hineinschrauben des Kolbens 5 in den Zylinder 3 aneinander anliegenden Flanken 26, 27 des Schraubgewindes 11 bzw. der Zähne 25 im wesentlichen senkrecht zu der Längsachse 6 verlaufend ausgebildet. Dadurch wird erreicht, daß die bei einem Hineinschrauben des Kolbens 5 entstehenden hohen Druckkräfte zwischen den Flanken 26 und 27 vollständig aufgenommen werden, ohne daß eine Kraftkomponente in einer Richtung senkrecht zur Längsachse auf die Flanken 27 wirkt, die ein Verschieben des Verriegelungselements 18 entgegen der Kraft der Feder 20 bewirken könnte. Ein ungewolltes Entkoppeln des Verriegelungselements 18 von dem Schaft 10 ist damit auch bei Auftreten eines sehr hohen Drucks ausgeschlossen.

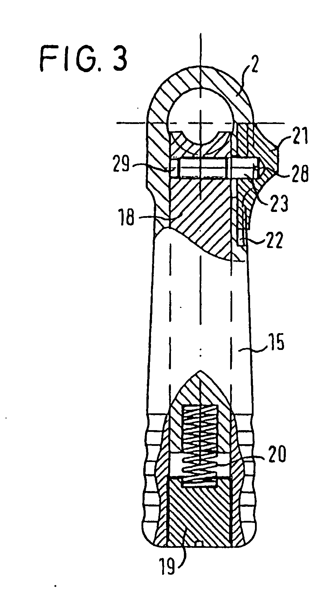

Aus dem Teilquerschnitt gemäß Fig. 3 ist zu erkennen, daß die Zähne 25 der Zahnstange 24 im Querschnitt teilringförmig ausgebildet sind und somit einen vergrößerten Kontaktbereich zu dem Schraubgewinde 11 schaffen. Dadurch ist gewährleistet, daß die zwischen den Zähnen 25 und dem Schraubgewinde 11 auftretende Kraft auf eine möglichst große Fläche verteilt wird, so daß ein Ausbrechen der Zähne 25 oder des Schraubgewindes 11 vermieden wird.

Weiterhin ist in Fig. 3 die Kopplung des Verriegelungselements 18 mit dem Entriegelungselements 21 über den Bolzen 23 erkennbar. Sowohl das Entriegelungselements 21 als auch das Verriegelungselement 18 besitzen dazu jeweils eine Bohrung 28, 29, in die der Bolzen 23 jeweils mit einem Ende eingreift. Auf diese Weise wird eine direkte Kopplung zwischen dem Entriegelungselement 21 und dem Verriegelungselement 18 geschaffen.

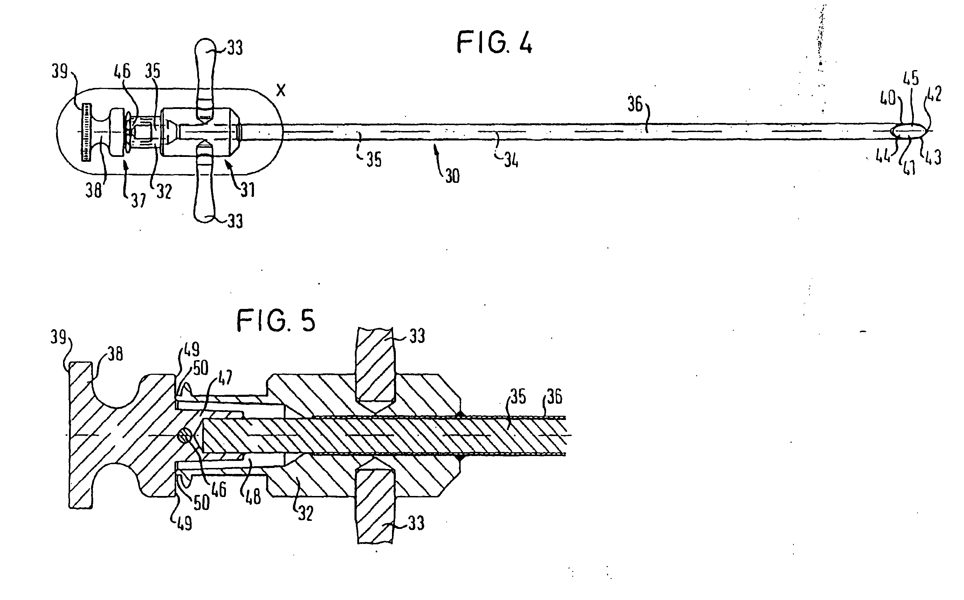

Fig. 4 zeigt eine erfindungsgemäß ausgebildete Kanüle, die beispielsweise mit einer Vorrichtung gemäß Fig. 1 verbunden werden kann. Die Kanüle 30 besitzt dazu an ihrem proximalen Ende 31 ein beispielsweise als große Luer Lock-Verbindung ausgebildetes Kopplungselement 32, das mit dem entsprechenden Kopplungselement 9 (siehe Fig. 1) abdichtend verbunden werden kann. Das Kopplungselement ist beispielsweise aus Metall hergestellt, um die beim Einschlagen der Kanüle auftretenden Kräfte aufnehmen zu können.

An dem proximalen Ende 31 der Kanüle 30 sind zwei sich radial nach au-ßen erstreckende, stiftförmige Angriffselemente 33 vorgesehen, über die die eingesetzte Kanüle 30 in einfacher Weise sowohl um ihre Längsachse 34 verdreht als auch in Richtung der Längsachse 34 aus dem Körper des Patienten wieder herausgezogen werden kann.

Weiterhin ist in Fig. 4 ein Mandrin 35 dargestellt, der in das Rohr 36 der Kanüle 30 eingesetzt ist und an seinem proximalen Ende 37 ein Abschlußelement 38 umfaßt. Das Abschlußeelement 38 ist zum einen zum Halten des Mandrins 35 beim Einsetzen in die Kanüle 30 und beim Herausziehen aus der Kanüle 30 verwendbar. Zum anderen kann das Abschlußeelement 38 dazu verwendet werden, daß die Kanüle 30, falls erforderlich, mit einem Eintriebsmittel, beispielsweise einem Hammer, an die gewünschte Position verbracht wird. Dazu ist das stirnseitige Ende des Abschlußelements 38 als Schlagfläche 39 ausgebildet.

An dem distalen Ende 40 der Kanüle 30 ist eine Öffnung 41 vorgesehen, die beispielsweise durch einen Schrägschnitt des Rohrs 36 erzeugbar ist. Durch diesen Schrägschnitt ist das distale Ende 40 der Kanüle 30 bezüglich ihrer Längsachse 34 asymmetrisch ausgebildet, wobei insbesondere die Spitze 42 der Kanüle 30 seitlich der Längsachse 34, d.h. bei der Darstellung gemäß Fig. 4 in einer Ebene hinter der Längsachse 34 zu liegen kommt.

Die die Umrandung der Öffnung 41 bildende Kante 43 des Rohrs 36 ist so geschliffen, daß diese Kante 43 eine Schneide bildet.

Der Mandrin 35 ist an seinem distalen Ende ebenfalls abgeschrägt ausgebildet und so innerhalb der Kanüle 30 angeordnet, daß die entsprechende Schrägfläche 44 mit der ebenfalls schräg angeordneten Austrittsfläche 45 der Öffnung 41 in Deckung ist. Um diese Deckung zu gewährleisten, ist an dem proximalen Ende 37 des Mandrins 35 eine Justiereinheit in Form eines Stiftes 46 vorgesehen, der in eine entsprechende Ausnehmung an dem Kopplungselement 32 eingreift und damit eine Verdrehsicherung zwischen dem Mandrin 35 und der Kanüle 30 bildet.

Der Stift 46 ist in der Detaildarstellung nach Fig. 5 näher zu erkennen. Ebenso ist aus dieser Darstellung die Anordnung des Mandrins 35 innerhalb des Rohrs 36 der Kanüle 30 erkennbar.

Das Abschlußelement 38 besitzt einen Ansatz 47, der in einen Hohlraum 48 in dem Kopplungselement 32 eingreift. Dabei ist der Durchmesser des Ansatzes 47 geringer als die lichte Weite des Hohlraums 48, so daß auch beim Einschlagen der Kanüle 30 mit einem Hammer trotz der hohen Schlagkräfte kein Verklemmen des Abschlußelements 38 und damit des Mandrins 35 mit dem Kopplungselement 32 erfolgen kann. Dadurch ist gewährleistet, daß der Mandrin 35 nach erfolgter Positionierung der Kanüle 30 problemlos entfernt werden kann.

Zur Übertragung der auf die Schlagfläche 39 des Abschlußelements 38 auftreffenden Schlagkräfte auf die Kanüle 30 stützt sich das Abschlußelement 38 über eine Abstützfläche 49 an dem stirnseitigen Ende 50 des Kopplungselements 32 ab.

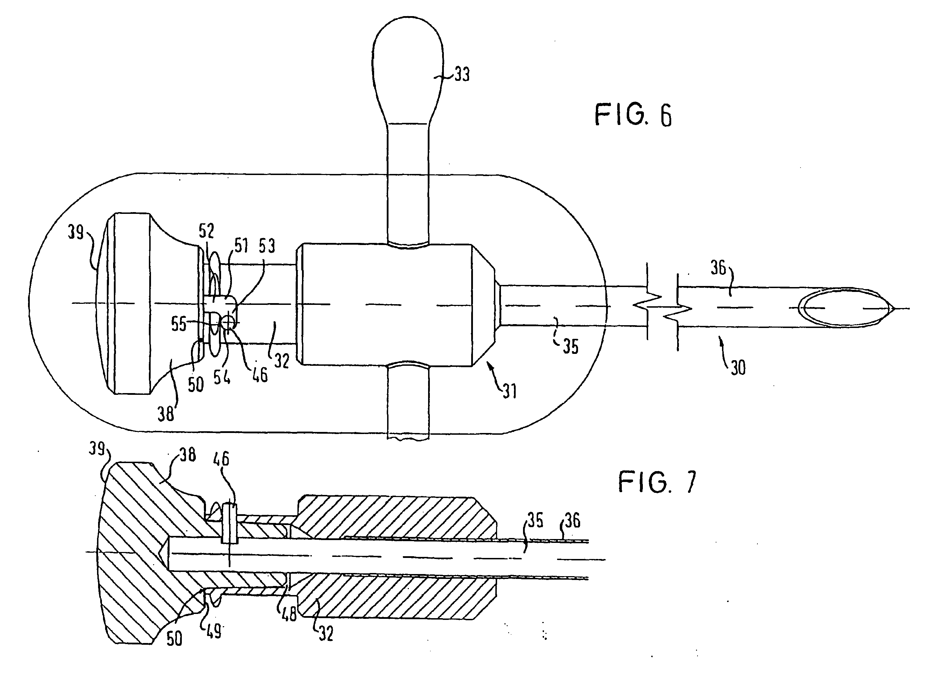

Während die Ausnehmung für den Stift 46 grundsätzlich beispielsweise als in axialer Richtung der Kanüle 30 verlaufender, gerader Schlitz ausgebildet sein kann, ist die Ausnehmung in den Fig. 6 und 7 als abgewinkelter oder L-förmiger Schlitz 51 ausgeführt. Der Schlitz 51 umfaßt einen in axialer Richtung der Kanüle 30 verlaufenden Längsabschnitt 52, der an der ringförmigen, stirnseitigen Fläche 50 des Kopplungselements 32 das offene Ende des Schlitzes 51 bildet, sowie einen in Umfangsrichtung des Kopplungselements 32 verlaufenden Querabschnitt 53, der im wesentlichen senkrecht zum Längsabschnitt 52 angeordnet ist.

Der Schlitz 51 bildet zusammen mit dem Stift 46 einen Bajonettverschluß, wobei im Bereich des freien Endes 54 des Schlitzes 51 eine zur stirnseitigen Fläche 50 des Kopplungselements 32 hin gelegene Vertiefung in Form einer Rastausnehmung 54 vorgesehen ist, in der der Stift 46 bei geschlossenem Bajonettverschluß, je nach Tiefe der Rastausnehmung 55, teilweise oder ganz zu liegen kommt. Auf diese Weise ist trotz des sich in Umfangsrichtung des Kopplungselements 32 erstreckenden Querabschnitts 53 eine Verdrehsicherheit des Mandrins 35 gegenüber der Kanüle 30 gewährleistet.

Die erfindungsgemäße Applikationsvorrichtung 1 sowie die erfindungsgemäß ausgebildete Kanüle werden wir folgt verwendet:

Zunächst wird die Kanüle 30 zusammen mit dem eingesetzten Mandrin 35 in den Körper des Patienten eingeführt, wobei dies im Bedarfsfall unter Zuhilfenahme eines Hammers erfolgt. Bei der Ausführungsform nach den Fig. 6 und 7 wird dabei die Kopplung zwischen dem Mandrin 35 und der Kanüle 30 über den durch den Stift 46 und den Schlitz 51 gebildeten Bajonettverschluß hergestellt. Durch den Bajonettverschluß wird verhindert, daß beim Einschlagen der Kanüle 30 aufgrund einer Federwirkung des Mandrins 35 dieser zurückgedrängt wird und teilweise aus der Kanüle 30 austritt. Ohne entsprechende Sicherung könnte der Mandrin 35 soweit zurückgedrängt werden, daß der Stift 46 aus seiner Längsführung austritt und dadurch die Verdrehsicherheit zwischen Mandrin 35 und Kanüle 30 nicht mehr gegeben ist.

Die jeweilige Position der Kanüle 30 wird beim Einbringen beispielsweise am CT (Computertomograph) verfolgt. Weicht die Position des distalen Endes 40 der Kanüle 30 von der gewünschten Position ab, so wird die Kanüle 30 über die Angriffselemente 33 so verdreht, daß die Spitze 42 in Richtung der gewünschten Position zu liegen kommt. Bei einem weiteren Einbringen der Kanüle 30 wird aufgrund der asymmetrischen Spitze 42 ein gewünschtes Auswandern dieser Spitze 42 in Richtung der gewünschten Position erfolgen.

Ist die Kanüle 30 korrekt positioniert, so wird der Mandrin 35 über das Abschlußelement 38 ergriffen und gegebenenfalls nach Lösen des Bajonettverschlusses aus der Kanüle 30 herausgezogen.

Anschließend wird der verwendete Knochenzement angerührt und über eine mit dem Kopplungselement 9 der Applikationsvorrichtung 1 verbundene Aufziehkanüle in das Innere 4 des Zylinders 3 aufgesogen. Dazu wird das Entriegelungselement entgegen der Kraft der Feder 20 so nach unten verschoben, daß die Zähne 25 außer Eingriff mit dem Schraubgewinde 11 kommen, so daß durch einfaches Zurückziehen des Kolbens 5 mit dem Schaft 11 der Knochenzement über die Aufziehkanüle in den Zylinder 3 gesogen wird.

Anschließend wird die Aufziehkanüle von der Applikationsvorrichtung 1 getrennt und diese mit der bereits in Position gebrachten erfindungsgemäßen Injektionskanüle 30 verbunden.

Im nächsten Verfahrensschritt wird wiederum bei entkoppeltem Verriegelungselement 18 der Kolben 5 mit dem Schaft 10 durch Druckbeaufschlagung der Angriffsfläche 14 des Betätigungselements 12 direkt in den Zylinder 1 hineinverschoben, wodurch das im Inneren 4 des Zylinder 3 angeordnete Zementmaterial über die Kanüle 30 in den Knochen injiziert wird. Durch die Injektion in das Knochenmaterial wird ein ständig wachsender Druck aufgebaut, bis dieser schließlich so hoch wird, daß ein weiteres Applizieren des Knochenzements durch Drücken auf die Angriffsfläche 14 nicht mehr möglich ist.

Zu diesem Zeitpunkt wird das Entriegelungselement 21 freigegeben, so daß das Verriegelungselement 18 durch die Kraft der Feder 20 in Richtung des Schafts 10 verschoben wird, bis die Zähne 25 mit dem Schraubgewinde 11 in Eingriff sind.

Anschließend kann durch Verdrehen des Schafts 10 über das Betätigungselement 12 der Druck im Inneren 4 des Zylinders 3 weiter erhöht werden, so daß der Kolben 5 weiter langsam in das Innere des Zylinders 1 verschoben wird.
Wird beispielsweise durch Beobachtung am Fluoroskop erkannt, daß der applizierte Knochenzement in eine ungewünschte Richtung strömt, kann diese Flußrichtung beispielsweise dadurch geändert werden, daß die Kanüle 30 über die Angriffselemente 33 so verdreht wird, daß die Öffnung 41 in die gewünschte Richtung zeigt.

Ist die aus der Öffnung 41 austretende Menge des Knochenzements aufgrund des hohen Drucks zu groß, so kann dieser Druck unverzüglich durch Verschieben des Entriegelungselements 21 und die dadurch erfolgende Entkopplung und Freigabe des Schafts 10 und des damit verbundenen Kolbens 5 abgebaut werden. Auf diese Weise wird verhindert, daß der Knochenzement an gefährliche Stellen innerhalb des Körpers appliziert wird.

Anschließend kann beispielsweise nach einer Neuorientierung der Kanüle 30 durch Verdrehen über die Angriffselemente 33 der Druck zunächst durch direktes Verschieben und anschließend, wie beschrieben, durch eine weiterführende Schraubbewegung wieder neu aufgebaut werden.

Nach vollständiger Applikation des Knochenzements kann die Applikationsvorrichtung 1 von der Kanüle 30 getrennt werden.

Nach Aushärten kann anschließend die Kanüle 30 leicht verkippt und gleichzeitig unter Zuhilfenahme der Angriffselemente 33 um ihre Längsachse 34 verdreht werden. Durch die schräge, geschliffene Kante 43 erfolgt dabei eine Abscherung des sich noch innerhalb des Rohrs 36 befindenden ausgehärteten Knochenzements, so daß bei einem anschließenden Herausziehen der Kanüle 30 dieses Material sicher zusammen mit der Kanüle 30 aus dem Körper entnommen wird.

### Bezugszeichenliste

- 1: Applikationsvorrichtung
- 2: Gehäuse
- 3: Zylinder
- 4: Inneres des Zylinders
- 5: Kolben
- 6: Längsachse
- 7: Abdichtungsmittel
- 8: Austrittsöffnung
- 9: Kopplungselement
- 10: Schaft
- 11: Schraubgewinde
- 12: Betätigungselement
- 13: Vertiefungen
- 14: Angriffsfläche
- 15: Handgriff
- 16: oberer Bereich des Handgriffs
- 17: Hohlraum
- 18: Verriegelungselement
- 19: Verschlußelement
- 20: Schraubenfeder
- 21: Entriegelungselement
- 22: Führungsausnehmung
- 23: Bolzen
- 24: Zahnstange
- 25: Zähne der Zahnstange (Gegenverzahnung)
- 26: Flanken
- 27: Flanken
- 28: Bohrung
- 29: Bohrung
- 30: Kanüle
- 31: proximales Endes der Kanüle
- 32: Kopplungselement
- 33: Angriffselemente
- 34: Längsachse der Kanüle
- 35: Mandrin
- 36: Rohr
- 37: proximales Ende des Mandrins
- 38: Abschlußelement
- 39: Schlagfläche
- 40: distales Ende des Mandrins
- 41: Öffnung
- 42: Spitze
- 43: Kante
- 44: Schrägfläche
- 45: Austrittsfläche
- 46: Stift
- 47: Ansatz
- 48: Hohlraum
- 49: Abstützfläche
- 50: stirnseitige Fläche des Kopplungselements
- 51: Schlitz
- 52: Längsabschnitt
- 53: Querabschnitt
- 54: freies Ende des Schlitzes 51
- 55: Rastausnehmung

## Patentansprüche

1. Vorrichtung zum Applizieren von Knochenzement mit einem Gehäuse (2), das einen Zylinder (3) zur Aufnahme des Knochenzements umfaßt, und mit einem in dem Zylinder (3) längsverschiebbar angeordneten Kolben (5), durch den der Knochenzement durch eine in dem Zylinder (5) ausgebildete Austrittsöffnung (8) herauspreßbar ist, wobei der Kolben (5) einen *Schaft (10) mit einem Schraubgewinde (11) umfasst und* zum Applizieren des Knochenzements unter hohem Druck durch eine Schraubbewegung in dem Zylinder (3) längsverschiebbar ist,
**dadurch gekennzeichnet,**
**dass** das Gehäuse (2) pistolenförmig mit einem Handgriff (15) ausgebildet ist,
**dass** der Handgriff (15) einen Hohlraum (17) umfasst,
**dass** in dem Hohlraum (17) ein Verriegelungselement (18) angeordnet ist, das in einer Richtung senkrecht zur Verschieberichtung des Kolbens (5) längsverschiebbar ist *und dessen zum Schaft (10) hin gelegenes Ende als Zahnstange (24) mit Zähnen (25) ausgebildet ist, die eine Gegenverzahnung für das Schraubgewinde (11) bilden,*
**dass** in dem Hohlraum (17) eine senkrecht zur Verschieberichtung des Kolbens (5) vorgespannte Feder (20) angeordnet ist,
**dass** die Feder (20) an dem Verriegelungselement (18) angreift und dieses zur Anlage gegen den Kolben (5) drängt, und
**daß** die Vorrichtung (1) durch Längsverschieben des Verriegelungselements (18) zwischen der Verschiebung des Kolbens (5) durch die Schraubbewegung und einer direkten Verschiebung in Längsrichtung ohne Schraubbewegung umstellbar ist.

2. Vorrichtung nach Anspruch 1,
**dadurch gekennzeichnet,**
**daß** der Kolben (5) einen Eingriffsabschnitt (10) mit einem Schraubgewinde (11) umfaßt, das in eine am Gehäuse (2) vorgesehene Gegenverzahnung (24) eingreift, so daß beim Verdrehen des Eingriffsabschnitts (10) die Längsverschiebung des Kolbens (5) erfolgt.

3. Vorrichtung nach Anspruch 2,
**dadurch gekennzeichnet,**
**daß** das Schraubgewinde (11) und die Gegenverzahnung (24) entkoppelbar sind.

4. Vorrichtung nach einem der Ansprüche 2 oder 3,
**dadurch gekennzeichnet,**
**daß** die Gegenverzahnung (24) das Schraubgewinde bereichsweise, insbesondere hinterschneidungsfrei umgreift.

5. Vorrichtung nach einem der vorhergehenden Ansprüche,
**dadurch gekennzeichnet,**
**daß** im entkoppelten Zustand der Kolben (5) im wesentlichen frei in dem Zylinder (3) längsverschiebbar ist.

6. Vorrichtung nach einem der Ansprüche 2 bis 5,
**dadurch gekennzeichnet,**
**daß** die beim Applizieren des Knochenzements unter Druck aneinander anliegenden Zahnflanken (26) der Gegenverzahnung (24) und/oder der Flanken (27) des Gewindeprofils des Schraubgewindes (11) einen Winkel von kleiner oder gleich ca. 90° mit der zur Verschieberichtung parallel verlaufenden Längsachse (6) des Eingriffsabschnitts (10) einschließen.

7. Vorrichtung nach einem der Ansprüche 2 bis 6,
**dadurch gekennzeichnet,**
**daß** die anderen Zahnflanken der Gegenverzahnung (24) und/oder die anderen Flanke des Gewindeprofils des Schraubgewindes (11) einen Winkel von mehr als ca. 90° mit der zur Verschieberichtung parallel verlaufenden Längsachse (6) des Eingriffsabschnitts (10) einschließen.

8. Vorrichtung nach einem der Ansprüche 2 bis 7,
**dadurch gekennzeichnet,**
**daß** der Kolben (5) und der Eingriffsabschnitt (10) einstückig ausgebildet sind.

9. Vorrichtung nach einem der Ansprüche 2 bis 7,
**dadurch gekennzeichnet,**
**daß** der Kolben (5) und der Eingriffsabschnitt (10) als separate Teile ausgebildet sind, die miteinander verbunden und insbesondere gegeneinander verdrehbar sind.

10. Vorrichtung nach einem der vorhergehenden Ansprüche,
**dadurch gekennzeichnet,**
**daß** der Zylinder (3) als Kreiszylinder ausgebildet ist.

11. Vorrichtung nach einem der vorhergehenden Ansprüche,
**dadurch gekennzeichnet,**
**daß** eine Kanüle (30) mit einer am distalen Ende (40) ausgebildeten Öffnung (41) und einem am proximalen Ende (31) vorgesehenen Kopplungsabschnitt (32) zum Ankoppeln an die Applikationsvorrichtung (1), vorgesehen ist,
**daß** das distale Ende (40) der Kanüle (30) asymmetrisch mit einer seitlich der Längsachse (34) liegenden Spitze (42) ausgebildet ist und daß am proximalen Ende (31) der Kanüle (30) seitlich nach au-ßen ragende Angriffselemente (33) vorgesehen sind, mit denen die Kanüle (30) sowohl um ihre Längsachse (34) verdrehbar als auch entlang ihrer Längsrichtung verschiebbar ist.

12. Vorrichtung nach Anspruch 11,
**dadurch gekennzeichnet,**
**daß** die Kanüle (30) an der Austrittsöffnung (8) des Zylinders (3) befestigbar ist.

13. Vorrichtung nach Anspruch 11 oder 12,
**dadurch gekennzeichnet,**
**daß** die die Umrandung der Öffnung (41) bildende Kante (43) des Kanülenendes (40) als Schneide geschliffen ist.

14. Vorrichtung nach einem der Ansprüche 11 bis 13,
**dadurch gekennzeichnet,**
**daß** sich die Durchtrittsfläche (45) der Öffnung (41) schräg zur Längsachse (34) der Kanüle (30) erstreckt.

15. Vorrichtung nach einem der Ansprüche 11 bis 14,
**dadurch gekennzeichnet,**
**daß** zwei Angriffselemente (33) vorgesehen sind, die sich bezüglich der Längsachse (34) der Kanüle (30) insbesondere gegenüberliegend angeordnet sind.

16. Vorrichtung nach einem der Ansprüche 11 bis 15,
**dadurch gekennzeichnet,**
**daß** die Angriffselemente (33) als radial nach außen vorstehende stiftförmige Elemente ausgebildet sind.

17. Vorrichtung nach einem der Ansprüche 11 bis 16,
**dadurch gekennzeichnet,**
**daß** in die Kanüle (30) ein Mandrin (35) einsetzbar ist und daß in dem Bereich des proximalen Endes (31) der Kanüle (30), insbesondere an dem Kopplungsabschnitt (32), ein Verbindungselement (51) zur Erzeugung einer lösbaren, drehfesten und/oder in axialer Richtung verschiebefesten Verbindung zwischen dem Mandrin (35) und der Kanüle (30) vorgesehenen ist.

18. Vorrichtung nach Anspruch 17,
**dadurch gekennzeichnet,**
**daß** an dem Mandrin (35), insbesondere im Bereich des proximalen Endes des Mandrins (35), ein mit dem Verbindungselement (51) zusammenwirkendes Gegenelement (46) vorgesehen ist.

19. Vorrichtung nach Anspruch 17 oder 18,
**dadurch gekennzeichnet,**
**daß** die Verbindung zwischen dem Mandrin (35) und der Kanüle (30) durch einen Bajonettverschluß gebildet wird.

20. Vorrichtung nach einem der Ansprüche 17 bis 19,
**dadurch gekennzeichnet,**
**daß** das Verbindungselement als schlitzförmige Ausnehmung (51), insbesondere als Nut oder Durchbrechung, und das Gegenelement als insbesondere stiftförmiger Ansatz (46) oder umgekehrt ausgebildet sind.

21. Vorrichtung nach Anspruch 20,
**dadurch gekennzeichnet,**
**daß** die Ausnehmung (51) zumindest einen in axialer Richtung der Kanüle (30) verlaufenden Längsabschnitt (52) umfaßt.

22. Vorrichtung nach Anspruch 21,
**dadurch gekennzeichnet,**
**daß** sich an den Längsabschnitt (52) ein in Umfangsrichtung der Kanüle (30) verlaufender Querabschnitt (53) der Ausnehmung (51) anschließt.

## Claims

1. An apparatus for the application of bone cement having a housing (2) which comprises a cylinder (3) for the reception of the bone cement and having a piston (5) which is arranged in a longitudinally displaceable manner in the cylinder (3) and by which the bone cement can be pressed out through an exit aperture (8) formed in the cylinder (3), wherein the piston (5) includes a shaft (10) having a screw thread (11) and is longitudinally displaceable in the cylinder (3) by a screw movement for the application of the bone cement under high pressure,
**characterized in that**
the housing (2) is designed in the form of a pistol having a handle (15);
**in that** the handle (15) includes a hollow space (17);
**in that** a locking element (17) is arranged in the hollow space (17) and is longitudinally displaceable in a direction perpendicular to the direction of displacement of the piston (5) and whose end disposed toward the shaft (10) is formed as a rack (24) having teeth (25) which form a mating toothed arrangement for the screw thread (11);
**in that** a spring (20) biased perpendicular to the direction of displacement of the piston (5) is arranged in the hollow space (17);
**in that** the spring (20) engages at the locking element (18) and urges it toward contact with the piston (5); and
**in that** the apparatus (1) can be switched between the displacement of the piston (5) by the screw movement and a direct displacement in the longitudinal direction without a screw movement by longitudinal displacement of the locking element (18).

2. An apparatus in accordance with claim 1,
**characterized in that**
the piston (5) comprises an engagement section (10) having a screw thread (11) that engages into a mating toothed arrangement (24) provided at the housing (2) such that the longitudinal displacement of the piston (5) takes place when the engagement section (10) is turned.

3. An apparatus in accordance with claim 2,
**characterized in that**
the screw thread (11) and the mating toothed arrangement (24) can be uncoupled.

4. An apparatus in accordance with one of the claims 2 or 3,
**characterized in that**
the mating toothed arrangement (24) engages around the screw thread regionally, in particular free of undercutting.

5. An apparatus in accordance with any one of the preceding claims,
**characterized in that**
the piston (5) can be longitudinally displaced in a substantially free manner in the cylinder (3) in the uncoupled state.

6. An apparatus in accordance with any one of the claims 2 to 5,
**characterized in that**
the tooth flanks (26) of the mating toothed arrangement (24) and/or of the flanks (27) of the thread section of the screw thread (11), which contact one another during the application of the bone cement under pressure, include an angle of less than or equal to approximately 90° with the longitudinal axis (6) of the engagement section (10) which extends parallel to the direction of displacement.

7. An apparatus in accordance with any one of the claims 2 to 6,
**characterized in that**
the other tooth flanks of the mating toothed arrangement (24) and/ or the other flank of the thread section of the screw thread (11) include an angle of more than approximately 90° with the longitudinal axis (6) of the engagement section (10) which extends parallel to the direction of displacement.

8. An apparatus in accordance with any one of the claims 2 to 7,
**characterized in that**
the piston (5) and the engagement section (10) are formed as one piece.

9. An apparatus in accordance with any one of the claims 2 to 7,
**characterized in that**
the piston (5) and the engagement section (10) are made as separate parts which are connected to one another and which are in particular rotatable with respect to one another.

10. An apparatus in accordance with any one of the preceding claims,
**characterized in that**
the cylinder (3) is formed as a circular cylinder.

11. An apparatus in accordance with any one of the preceding claims,
**characterized in that**
a cannula (30) is provided having an aperture (41) formed at the distal end (40) and a coupling section (32) provided at the proximal end (31) for the coupling to the application apparatus (1);
**in that** the distal end (40) of the cannula (30) is formed asymmetrically with a tip (42) disposed to the side of the longitudinal axis (34) and **in that** handling elements (33) projecting outwardly to the side are provided at the proximal end (31) of the cannula (30) with which the cannula (30) can both be rotated about its longitudinal axis (34) and displaced about its longitudinal direction.

12. An apparatus in accordance with claim 11,
**characterized in that**
the cannula (30) can be fastened to the exit aperture (8) of the cylinder (3).

13. An apparatus in accordance with one of the claims 11 or 12,
**characterized in that**
the edge (43) of the cannula end (40) forming the border of the aperture (41) is ground as a cutting edge.

14. An apparatus in accordance with any one of the claims 11 to 13,
**characterized in that**
the passage area (45) of the aperture (41) extends obliquely to the longitudinal axis (34) of the cannula (30).

15. An apparatus in accordance with any one of the claims 11 to 14,
**characterized in that**
two handling elements (33) are provided which are arranged with respect to the longitudinal axis (34) of the cannula (30), in particular opposite thereto.

16. An apparatus in accordance with any one of the claims 11 to 15,
**characterized in that**
the holding elements (33) are formed as pin-like elements projecting radially outwardly.

17. An apparatus in accordance with any one of the claims 11 to 16,
**characterized in that**
a mandrin (35) can be inserted into the cannula (30); and **in that** a connecting element (51) is provided in the region of the proximal end (31) of the cannula (30), in particular at the coupling section (32), to generate a connection between the mandrin (35) and the cannula (30) which is releasable, rotationally fixed and/or displaceably fixed in the axial direction.

18. An apparatus in accordance with claim 17,
**characterized in that**
a mating element (46) cooperating with the connecting element (51) is provided at the mandrin (35), in particular in the region of the proximal end of the mandrin (35).

19. An apparatus in accordance with claim 17 or claim 18,
**characterized in that**
the connection between the mandrin (35) and the cannula (30) is formed by a bayonet fastening.

20. An apparatus in accordance with any one of the claims 17 to 19,
**characterized in that**
the connecting element is formed as a slot-like recess (51), in particular as a groove or breakthrough, and the mating element is formed in particular as a pin-like lug (46) or vice versa.

21. An apparatus in accordance with claim 20,
**characterized in that**
the recess (51) comprises at least one longitudinal section (52) extending in the axial direction of the cannula (30).

22. An apparatus in accordance with claim 21,
**characterized in that**
a cross-section (53) of the recess (51) extending in the peripheral direction of the cannula (30) adjoins the longitudinal section (52).

## Revendications

1. Dispositif pour appliquer du ciment osseux, comprenant un boîtier (2) qui inclut un cylindre (3) pour recevoir le ciment osseux et comprenant un piston (5) agencé avec faculté de translation longitudinale dans le cylindre (3), au moyen duquel le ciment osseux peut être pressé à travers une ouverture de sortie (8) réalisée dans le cylindre (3), dans lequel le piston (5) comprend une tige (10) avec un pas de vis (11) et est susceptible d'être déplacé en translation longitudinale par un mouvement de vissage dans le cylindre (3) pour appliquer le ciment osseux sous haute pression,
**caractérisé en ce que**
le boîtier (2) est réalisé sous forme de pistolet avec une poignée (15), **en ce que** la poignée (15) inclut une cavité (17),
**en ce qu'**un élément de verrouillage (18) est agencé dans la cavité (17), lequel est susceptible de se déplacer en translation longitudinale dans une direction perpendiculaire à la direction de translation du piston (5), et dont l'extrémité disposée vers la tige (10) est réalisée sous forme de crémaillère (24) avec des dents (25) qui forment une denture antagoniste pour le pas de vis (11),
**en ce qu'**un ressort (20) précontraint perpendiculairement à la direction de translation du piston (5) est agencé dans la cavité (17),
**en ce que** le ressort (20) engage l'élément de verrouillage (38) et force de celui-ci en appui contre le piston (5), et
**en ce que** le dispositif (1) est susceptible d'être converti, par translation longitudinale de l'élément de verrouillage (18), entre une translation du piston (5) par le mouvement de vissage et une translation directe en direction longitudinale sans mouvement de vissage.

2. Dispositif selon la revendication 1,
**caractérisé en ce que** le piston (9) comprend un tronçon d'engagement (10) avec un pas de vis (11) qui s'engage dans une denture antagoniste (24) prévue sur le boîtier (2) de telle manière que la translation longitudinale du piston (5) se produit par rotation du tronçon d'engagement (10).

3. Dispositif selon la revendication 2,
**caractérisé en ce que** le pas de vis (11) et la denture antagoniste (24) peuvent être découplés.

4. Dispositif selon l'une des revendications 2 ou 3,
**caractérisé en ce que** la denture antagoniste (24) entoure le pas de vis par secteur, en particulier en étant dépourvue de contre-dépouille.

5. Dispositif selon l'une des revendications précédentes,
**caractérisé en ce que**, dans l'état découplé, le piston (5) est déplaçable sensiblement librement en translation longitudinale dans le cylindre (3).

6. Dispositif selon l'une des revendications 2 à 5,
**caractérisé en ce que** les flancs des dents (26) de la denture antagoniste (24) et/ou les flancs (27) du profil du pas de vis (11), appliqués sous pression les uns contre les autres lors de l'application du ciment osseux, définissent un angle inférieur ou égal à environ 90° avec l'axe longitudinal (6) du tronçon d'engagement (10) qui s'étend parallèlement à la direction de translation.

7. Dispositif selon l'une des revendications 2 à 6,
**caractérisé en ce que** les autres flancs des dents de la denture antagoniste (24) et/ou les autres flancs du profil du pas de vis (11) définissent un angle supérieur à environ 90° avec l'axe longitudinal (6) du tronçon d'engagement (10) qui s'étend parallèlement à la direction de translation.

8. Dispositif selon l'une des revendications 2 à 7,
**caractérisé en ce que** le piston (5) et le tronçon d'engagement (10) sont réalisés d'une seule pièce.

9. Dispositif selon l'une des revendications 2 à 7,
**caractérisé en ce que** le piston (5) et le tronçon d'engagement (10) sont réalisés comme des pièces séparées qui sont reliées l'une à l'autre et qui sont en particulier capables de tourner l'une par rapport à l'autre.

10. Dispositif selon l'une des revendications précédentes,
**caractérisé en ce que** le cylindre (3) est réalisé sous forme de cylindre à base circulaire.

11. Dispositif selon l'une des revendications précédentes,
**caractérisé en ce qu'**il est prévu une canule (30) avec une ouverture (41) réalisée à l'extrémité distale (40) et un tronçon d'accouplement (32) prévu à l'extrémité proximale (31) pour l'accouplement au dispositif d'application (1),
**en ce que** l'extrémité distale (40) de la canule (30) est réalisée asymétrique avec une pointe (42) située latéralement par rapport à l'axe longitudinal (34), et
**en ce qu'**il est prévu à l'extrémité proximale (31) de la canule (30) des éléments d'engagement (33) en saillie latéralement vers l'extérieur, avec lesquels la canule (30) peut être aussi bien mise en rotation autour de son axe longitudinal (34) que mise en translation le long de sa direction longitudinale.

12. Dispositif selon la revendication 11,
**caractérisé en ce que** la canule (30) peut être fixée à l'ouverture de sortie (8) du cylindre (3).

13. Dispositif selon la revendication 11 ou 12,
**caractérisé en ce que** l'arête (43) qui forme la bordure de l'ouverture (41) à l'extrémité de la canule (40) est affûtée à la manière d'un tranchant.

14. Dispositif selon l'une des revendications 11 à 13,
**caractérisé en ce que** la surface de passage (45) de l'ouverture (41) s'étend en oblique par rapport à l'axe longitudinal (34) de la canule (30).

15. Dispositif selon l'une des revendications 11 à 14,
**caractérisé en ce qu'**il est prévu deux éléments d'engagement (33) qui sont agencés en particulier en opposition par rapport à l'axe longitudinal (34) de la canule (30).

16. Dispositif selon l'une des revendications 11 à 15,
**caractérisé en ce que** les éléments d'engagement (33) sont réalisés comme des éléments en forme de tige en saillie radialement vers l'extérieur.

17. Dispositif selon l'une des revendications 11 à 16,
**caractérisé en ce qu'**un mandrin (35) peut être introduit dans la canule (30) et **en ce que**, dans la région de l'extrémité proximale (31) de la canule (30), en particulier au niveau du tronçon d'accouplement (32), il est prévu un élément de liaison (31) pour engendrer une liaison détachable, solidaire en rotation et/ou solidaire en translation en direction axiale, entre le mandrin (35) et la canule (30).

18. Dispositif selon la revendication 17,
**caractérisé en ce qu'**il est prévu un élément antagoniste (46), qui coopère avec l'élément de liaison (51), sur le mandrin (35) et en particulier dans la région de l'extrémité proximale du mandrin (35).

19. Dispositif selon la revendication 17 ou 18,
**caractérisé en ce que** la liaison entre le mandrin (35) et la canule (30) est formée par une fermeture à baïonnette.

20. Dispositif selon l'une des revendications 17 à 19,
**caractérisé en ce que** l'élément de liaison est réalisé comme un évidement (51) en forme de fente, en particulier comme une rainure ou comme une traversée, et **en ce que** l'élément antagoniste est réalisé comme un talon (46) en particulier en forme de tige, ou inversement.

21. Dispositif selon la revendication 20,
**caractérisé en ce que** l'évidement (51) comprend au moins un tronçon longitudinal (52) qui s'étend en direction axiale de la canule (30).

22. Dispositif selon la revendication 21,
**caractérisé en ce que** le tronçon longitudinal (52) est suivi d'un tronçon transversal (53), qui s'étend en direction périphérique de la canule (30), de l'évidement (51).
